# EUROPÄISCHE PATENTSCHRIFT

(11) **EP 0 519 435 B1**
(45) Veröffentlichungstag und Bekanntmachung des Hinweises auf die Patenterteilung: **15.03.1995**
(21) Anmeldenummer: 92110246.3
(22) Anmeldetag: 17.06.1992
(51) Int. Cl.: C07C 67/055, C07C 69/15, B01J 37/00

(54) **Trägerkatalysator, Verfahren zu seiner Herstellung, sowie seine Verwendung zur Herstellung von Vinylacetat**
Supported catalyst, method for the preparation thereof, and use for the production of vinylacetate
Catalyseur supporté, son procédé de préparation ainsi que son utilisation pour la préparation d'acétate de vinyle

(30) Priorität: 21.06.1991 DE 4120492
(43) Veröffentlichungstag der Anmeldung: 23.12.1992
(73) Patentinhaber: HOECHST AKTIENGESELLSCHAFT, 65926 Frankfurt am Main (DE)
(72) Erfinder: Wirtz, Peter, Dr., W-6240 Königstein/Ts. (DE); Wörner, Karl-Fred, Dr., W-6236 Eschborn (DE); Wunder, Friedrich, Dr., W-6234 Hattersheim/M. (DE); Eichler, Klaus, Dr., W-6236 Eschborn (DE); Roscher, Günter, Dr., W-6233 Kelkheim (Taunus) (DE)

(56) Entgegenhaltungen:
- EP-A- 0 330 853
- EP-A- 0 403 950
- EP-A- 0 431 478
- DE-B- 1 249 255

## Beschreibung

Es ist bekannt, daß man Ethylen in der Gasphase mit Essigsäure und Sauerstoff oder sauerstoffhaltigen Gasen an Katalysatoren zu Vinylacetat umsetzen kann. Geeignete Katalysatoren enthalten Palladium und/oder dessen Verbindungen und Alkaliverbindungen, sowie zusätzlich Cadmiumverbindungen und/oder Gold und/oder dessen Verbindungen. Bevorzugte Alkaliverbindungen sind Kaliumverbindungen (US-PS 3 939 199, US-PS 4 668 819). Diese aktiven Komponenten werden auf Träger aufgebracht, wobei als Trägermaterial im allgemeinen Kieselsäure oder Aluminiumoxid verwendet werden.

In der US-PS 4 048 096 und der US-PS 3 775 342 wird eine Sonderform der Edelmetallverteilung beschrieben, bei der die Edelmetalle in einer Schale auf den Trägerteilchen vorliegen, während der Kern der Teilchen weitgehend frei von Edelmetallen ist. Durch diese Maßnahme erhält man eine erhöhte spezifische Leistung (g Vinylacetat/g Edelmetall). Die Edelmetallverteilung in Schalenform erreicht man durch Imprägnierung und anschließendes Ausfällen der Edelmetalle mit alkalischen Verbindungen.

Nach US-PS 3 939 199 soll das Gesamtporenvolumen eines sehr leistungsfähigen Trägers bei 0,4 - 1,2 ml/g liegen, und weniger als 10 % dieses Volumens sollen von "Mikroporen" mit einem Porendurchmesser unter 30 Å (Angström) gebildet werden. Solche Träger können aus aerogenem SiO₂ oder einem aerogenen SiO₂-Al₂O₃-Gemisch hergestellt werden, welches in Form von glasigen Mikrokugeln vorliegt, die beispielsweise durch Flammenhydrolyse von Siliciumtetrachlorid oder eines Siliciumtetrachlorid-Aluminiumtrichlorid-Gemisches in einer Knallgasflamme hergestellt werden können. Im Handel sind diese Mikrokugeln unter dem Namen ®Aerosil oder ®Cabosil erhältlich.

In der DE-OS 39 19 524 wird ein Träger der eben genannten Art beschrieben, der aus SiO₂ oder SiO₂-Al₂O₃-Gemisch mit einer Oberfläche von 50 - 250 m²/g und einem Porenvolumen von 0,4 - 1,2 ml/g besteht und dessen Teilchen eine Korngröße von 4 bis 9 mm haben, wobei 5 bis 20 % des Porenvolumens des Trägers von Poren mit Radien von 200 bis 3000 Å und 50 bis 90 % des Porenvolumens von Poren mit Radien von 70 bis 100 Å gebildet wird.

Aus den Mikrokugeln werden dann vorteilhafterweise gemäß EP-A-0 431 478 unter Zusatz von einem oder mehreren Carboxylaten von Li, Mg, Al, Zn, Fe oder Mn als Bindemitteln und unter Zusatz von organischen Füllstoffen (wie Zucker, Harnstoff, höheren Fettsäuren, längerkettigen Paraffinen, mikrokristalliner Cellulose) und Gleitmitteln (wie Kaolin, Graphit, Metallseifen), Formkörper, d.h. geformte Trägerteilchen, z.B. durch Tablettieren oder Extrudieren, hergestellt. Anschließend werden die Formkörper in O₂-haltigen Gasen geglüht.

Versuche, auf diesen mit einem Bindemittel gepreßten Formkörpern durch Imprägnierung mit Edelmetallen and anschließende Behandlung mit einer alkalischen Verbindung eine Edelmetall-Schale zu erzeugen, schlagen fehl. Man erhält stattdessen eine homogene Edelmetall-Verteilung im gesamten Formkörper.

Es hat sich nun überraschenderweise herausgestellt, daß man eine schalenförmige Edelmetall-Verteilung erhält, wenn man die Formkörper vor der Imprägnierung mit Edelmetall solange mit einer Säure wäscht, bis keine weiteren Kationen des Bindemittels (Li, Mg, Al, Zn, Fe oder Mn) mehr ausgewaschen werden.

Gegenstand der Erfindung ist ein Verfahren zur Herstellung eines Katalysators, der Palladium und/oder dessen Verbindungen und Alkaliverbindungen, sowie zusätzlich Cadmiumverbindungen und/oder Gold und/oder dessen Verbindungen auf Trägerteilchen enthält, die aus SiO₂ oder einem SiO₂-Al₂O₃-Gemisch mit Hilfe eines Bindemittels, bestehend aus einem oder mehreren Li-, Mg-, Al-, Zn-, Fe- oder Mn-Salzen einer C₂-C₂₆Carboxylsäure, gepreßt und anschließend in sauerstoffhaltigen Gasen bei 500 - 900°C für eine Dauer von 0,25 - 5 Stunden geglüht wurden und danach eine Oberfläche von 50 - 250 m²/g und ein Porenvolumen von 0,4 - 1,2 ml/g bei einer Teilchengröße von 1 - 15 mm besitzen, wobei 5 - 20 % des Porenvolumens von Poren mit einem Radius von 200 - 3000 Å und 50 - 90 % des Porenvolumens von Poren mit einem Radius von 70 - 100 Å gebildet wird, das dadurch gekennzeichnet ist, daß man die geglühten Trägerteilchen
a) mit einer Säure wäscht, die nicht mit SiO₂ oder SiO₂-Al₂O₃-Gemischen reagiert, bis von den Trägerteilchen keine weiteren Kationen des beim Pressen der Trägerteilchen eingesetzten Bindemittels abgegeben werden;
b) dann die Trägerteilchen mit Palladium, sowie Gold oder Cadmium imprägniert;
c) dann die imprägnierten Trägerteilchen mindestens solange mit einer Lösung einer Base in Kontakt bringt, bis die Dicke der dadurch auf den Trägerteilchen erzeugten Edelmetallschale sich nicht mehr wesentlich ändert;
d) dann die Trägerteilchen mit einer Alkaliverbindung imprägniert.

Weitere Gegenstände der Erfindung sind ein auf diese Weise hergestellter Trägerkatalysator, sowie dessen Verwendung zur Herstellung von Vinylacetat in der Gasphase aus Ethylen, Essigsäure und Sauerstoff oder sauerstoffhaltigen Gasen.

Wegen der auf diese Weise erreichten Verteilung der Edelmetalle in Schalenform sind diese leichter für die Reaktion zugänglich, wodurch bessere spezifische Leistungen als bei homogener Verteilung erreicht werden. Hierdurch kann entweder bei gleicher Edelmetallmenge des Katalysators mehr Vinylacetat produziert werden als bei homogener Verteilung oder man kann bei gleicher Produktionsmenge den Edelmetallgehalt des Katalysators senken und somit Kosten einsparen. Besonders überraschend ist hierbei, daß trotz der Säurewäsche, bei der das Bindemittel fast vollständig entfernt wird, die mechanische Stabilität der Träger nicht leidet.

Der Gehalt an Palladium auf dem Schalenkatalysator liegt im allgemeinen bei 0,5 - 2,5 Gew.-%, vorzugsweise bei 0,7 - 1,8 Gew.-%, insbesondere bei 1,0 - 1,6 Gew.-%, bezogen auf die Gesamtmasse des Trägerkatalysators. Falls zusätzlich Gold aufgebracht wird, liegt dessen Anteil bei 0,2 - 0,7 Gew.-%, bezogen auf die Gesamtmasse des Trägerkatalysators.

Die als Aktivatoren eingesetzten Alkaliverbindungen werden im allgemeinen in einem Anteil von 0,5 - 5 Gew.-%, bezogen auf die Gesamtmasse des Katalysators aufgebracht.

Falls Cadmium als zusätzlicher Aktivator benutzt wird, liegt der Anteil bei 0,5 - 5 Gew.-%, bezogen auf die Gesamtmasse des Katalysators.

Die angegebenen Prozentzahlen betreffen stets die auf dem Katalysator anwesenden Mengen der Elemente Palladium, Gold, Alkalimetall, Cadmium; etwaige Anionen werden nicht mitgerechnet.

Bevorzugt sind folgende Katalysatoren:
Palladium/Cadmium/Kalium sowie Palladium/Gold/Kalium, wobei Palladium bzw. Gold als Metalle oder Verbindungen auf dem fertigen Katalysator vorliegen können.

Die Herstellung der Schalen-Katalysatoren gliedert sich in folgende Schritte:
Zunächst werden glasige Mikrokugeln, beispielsweise durch Flammenhydrolyse von Siliciumtetrachlorid oder eines Siliciumtetrachlorid-Aluminiumtrichlorid-Gemisches in einer Knallgasflamme hergestellt (US-PS 3 939 199). Besonders geeignet sind Mikrokugeln mit einer Oberfläche von 150 - 250 m²/g, die aus mindestens 95 Gew.-% SiO₂ und höchstens 5 Gew.-% Al₂O₃ bestehen.

Aus den Mikrokugeln werden dann unter Zusatz von einem oder mehreren C₂-C₂₆-Carboxylaten von Li, Mg, Zn, Al, Fe oder Mn als Bindemitteln und unter Zusatz von organischen Füllstoffen (wie Zucker, Harnstoff, höheren Fettsäuren, längerkettigen Paraffinen, mikrokristalliner Cellulose) und Gleitmitteln (wie Kaolin, Graphit, Metallseifen) Formkörper hergestellt. Bevorzugte Bindemittel sind Mg-, Al-, Li- oder Fe-Salze, insbesondere Mg- oder Al-Salze, von C₅-C₂₂-Carboxylsäuren, vor allem aber von C₁₀-C₂₀-Carboxylsäuren. Das oder die Carboxylate werden im allgemeinen in solchen Mengen eingesetzt, daß die Summe der Mengen von Li, Mg, Zn, Al, Fe, Mn (als Elemente gerechnet) 0,1 bis 5 Gew.-%, bezogen auf das Trägermaterial, beträgt, vorzugsweise 0,3 bis 1,5 Gew.-%. Anschließend werden die Formkörper zur Entfernung des mit diesen Zusätzen eingebrachten Kohlenstoffs in O₂-haltigen Gasen bei etwa 500 - 900°C für etwa 0,25 - 5 Stunden geglüht. Die Oberfläche des Trägers, sein Porenvolumen und der Anteil des Porenvolumens, der von Poren eines bestimmten Radius gebildet wird (Porenradienverteilung), wird von der Art der Verformung, der Temperatur und Dauer des Glühens, den relativen Mengen an Bindemittel, Füllstoffen, Gleitmitteln und Mikrokugeln sowie durch die Oberfläche dieser Mikrokugeln bestimmt. Diese Parameter sind so zu wählen, daß die Formkörper nach dem Glühen eine Oberfläche von 50 - 250 m²/g und ein Porenvolumen von 0,4 - 1,2 ml/g bei einer Teilchengröße von 1 - 15 mm besitzen, wobei 5 - 20 % des Porenvolumens von Poren mit einem Radius von 200 - 3000 Å und 50 - 90 % des Porenvolumens von Poren mit einem Radius von 70 - 100 Å gebildet wird.

Die Teilchengröße liegt bevorzugt bei 4 - 9 mm, besonders bevorzugt bei 5 - 7 mm, wobei als Formkörper Kugeln, Tabletten oder anders gestaltete Teilchen benutzt werden können.

Zur Säurewäsche der geglühten Formkörper (d.h. der geglühten und gepreßten Trägerteilchen) können alle Säuren benutzt werden, die nicht mit SiO₂ bzw. SiO₂-Al₂O₃-Gemischen reagieren und deren Kationen - falls diese desaktivierend wirken (wie Sulfat oder Chlorid) - durch Auswaschen vom Träger entfernt werden können.

Bevorzugte Säuren sind Mineralsäuren, wie Salzsäure, Schwefelsäure, Phosphorsäure oder Salpetersäure, besonders bevorzugt ist Salzsäure. Die Säuren können sowohl konzentriert als auch verdünnt eingesetzt werden. Es können auch Mischungen verschiedener Säuren benutzt werden. Die Säurewäsche wird solange durchgeführt, bis keine weiteren Kationen des Bindemittels abgegeben werden. Dies läßt sich dadurch erkennen, daß man den Träger nach einer gewissen Zeit aus der Säure entfernt und dann in frischer Säure wäscht; wenn in dieser Säure keine Kationen des Bindemittels mehr nachweisbar sind, war die Behandlungsdauer ausreichend. Die Dauer der Säurebehandlung ist unter anderem abhängig von der Art der Säure und vom Grad der Verdünnung; allgemein erfordern verdünnte Säuren eine längere Behandlungsdauer. Bevorzugt werden 5 - 50 %ige Säuren, besonders bevorzugt
10 - 20 %ige Säuren. Außerdem spielen auch die Art und Menge des Bindemittels und die Temperatur bei der Säurewäsche eine Rolle. Für die besonders bevorzugte Ausführung der Säurewäsche mit 10 - 20 %iger Salzsäure ist im allgemeinen eine Behandlungsdauer von 10 - 14 Stunden ausreichend. Die Menge der eingesetzten Säure wird vorzugsweise so bemessen, daß der Katalysator vollständig mit Flüssigkeit überdeckt ist.

Nach der Säurewäsche werden die Anionen der Säure ausgewaschen, falls sie für den Katalysator schädlich wären (wie Chlorid- oder Sulfationen); am einfachsten geschieht dies dadurch, daß man den Träger so lange mit fließendem destilliertem Wasser wäscht, bis nach gängigen Analysemethoden (z.B. Fällung mit Silbernitrat für Chlorid oder Fällung mit Bariumchlorid für Sulfat) keine Anionen mehr nachgewiesen werden. Vorzugsweise wird aber auch bei Säuren mit für die Katalyse unschädlichen Anionen eine Wasserwäsche durchgeführt.

Vor der nun folgenden Imprägnierung des Trägers mit Palladium sowie Gold oder Cadmium wird der Träger vorzugsweise getrocknet. Die Imprägnierung erfolgt vorzugsweise dadurch, daß in einem Lösungsmittel gelöste Salze der genannten Metalle zum Trägermaterial zugegeben werden. Als Lösungsmittel sind besonders Wasser oder Alkohole, wie Methanol oder Ethanol geeignet. Die Menge des Lösungsmittels entspricht zweckmäßigerweise dem integralen Porenvolumen des Trägers.

Als Verbindungen des Palladiums kommen alle Salze und Komplexe in Betracht, die löslich sind und im fertigen (wenn nötig, wie oben erläutert, gewaschenen) Katalysator keine desaktivierenden Stoffe hinterlassen. Besonders geeignet sind das Chlorid oder andere lösliche und einer Fällung als Oxid(hydrat) zugänglichen Salze wie Nitrat, Oxalat und Sulfat.

Als Goldverbindungen kommen besonders Goldchlorid oder die löslichen Salze der Tetrachlorgoldsäure in Betracht.

Als Cadmiumverbindungen kommen die Verbindungen in Frage, die löslich sind, z.B. Carboxylat, Oxid, Hydroxid, Carbonat, Chlorid, Citrat, Tartrat, Nitrat, Acetylacetonat, Acetoacetat.

Nunmehr folgt die Basenbehandlung des imprägnierten Trägers, der vorzugsweise vorher wieder getrocknet wird. Als Basen kommen besonders Alkalihydroxide, Alkalisilikate und Alkalicarbonate in Frage, bevorzugt sind Alkalihydroxide, besonders bevorzugt Kalium- und Natriumhydroxid. Auch Mischungen verschiedener Basen sind einsetzbar. Die Base sollte in einem Lösungsmittel gelöst sein, in dem auch die bei der Imprägnierung eingesetzten Pd- und Au- bzw. Cd-Verbindungen löslich sind; in Frage kommen z.B. Wasser und Alkohole. Um bei der Basenbehandlung Edelmetallverluste zu vermeiden, sollte das Volumen des Lösungsmittels vorzugsweise dem integralen Porenvolumen des Trägers entsprechen; es können jedoch auch größere Mengen an Lösungsmittel eingesetzt werden. Die notwendige Menge an Base ergibt sich aus der stöchiometrisch berechneten Menge an Hydroxidionen, die zur Überführung des Palladiums sowie des Golds oder Cadmiums in die Hydroxide benötigt werden; es erweist sich von Vorteil, einen Überschuß an Base zu verwenden, z.B. 100 - 200 % der stöchiometrisch benötigten Menge, bevorzugt 105 - 150 % der stöchiometrisch benötigten Mengen, besonders bevorzugt 110 - 140 % der stöchiometrisch benötigten Menge. Der Träger muß mindestens solange in Kontakt mit der Base bleiben, bis die Dicke der erzeugten Edelmetallschale sich nicht mehr wesentlich ändert. Dies läßt sich einfach feststellen, indem nach gewissen Zeitabständen Katalysatorteilchen entnommen und aufgeschnitten werden. Die Dauer der Behandlung mit der Base ist von deren Konzentration und der Umgebungstemperatur abhängig, bevorzugt wird bei Raumtemperatur gearbeitet. Im bevorzugten Fall des Einsatzes von Kalium- bzw. Natriumhydroxid in Wasser ist im allgemeinen eine Behandlungsdauer von etwa 6 Stunden ausreichend; eine längere Behandlung ist jedoch im allgemeinen unschädlich. Falls die bei der Palladium- und Gold- oder Cadmium-Imprägnierung eingesetzten Salze für den Katalysator schädliche Bestandteile enthielten (z.B. Chlorid oder Sulfat), wird der Katalysator nun so lange mit fließendem destilliertem Wasser gewaschen, bis diese desaktivierenden Stoffe nicht mehr nachweisbar sind.

Man kann den Katalysator nun reduzieren, z.B. mit einer Lösung von Hydrazinhydrat oder durch Überleiten reduzierender Gase wie Ethylen oder Methanol bei Raumtemperatur oder erhöhter Temperatur, wobei vorteilhafterweise das reduzierende Gas mit einem Inertgas wie Stickstoff verdünnt wird. Die Menge des Reduktionsmittels richtet sich nach der Menge des Palladiums und gegebenenfalls des eingesetzten Goldes; das Reduktionsäquivalent soll mindestens das 1 - 1,5-fache des Oxydationsäquivalents betragen, jedoch schaden größere Mengen nicht.

Nach der Basenbehandlung, oder gegebenenfalls nach der Reduktion, wird die Alkaliverbindung auf den Katalysator aufgebracht; vorher wird vorzugsweise getrocknet. Die Alkaliverbindung wird in einem geeigneten Lösungsmittel, z.B. Wasser oder Alkohol gelöst, wobei die Lösungsmittelmenge vorzugsweise dem integralen Porenvolumen des zu imprägnierenden Trägermaterials entspricht. Als Alkaliverbindungen können Carboxylate, z.B. Kaliumacetat, Natriumacetat oder Natriumpropionat eingesetzt werden. Geeignet sind auch andere Alkalisalze, wie Hydroxide, Oxide oder Carbonate, sofern sie unter den Reaktionsbedingungen in die Acetate überführt werden. Vorzugsweise setzt man Kaliumverbindungen ein, insbesondere Kaliumacetat.

Der Katalysator sollte vorzugsweise vor dem Einfüllen in den Reaktor getrocknet werden.

Die Herstellung des Vinylacetats erfolgt im allgemeinen durch Leiten von Essigsäure, Ethylen und Sauerstoff oder sauerstoffhaltigen Gasen bei Temperaturen von 100 - 220°C, vorzugsweise 120 - 200°C, und bei Drücken von 1 - 25 bar, vorzugsweise 1 - 20 bar, über den fertigen Katalysator, wobei nicht umgesetzte Komponenten im Kreis geführt werden können. Aufgrund der bekannten Zündgrenzen sollte die Sauerstoffkonzentration, bezogen auf das essigsäurefreie Gemisch, 10 Vol.-% nicht überschreiten. Unter Umständen ist eine Verdünnung mit inerten Gasen vorteilhaft, geeignet sind z.B. Stickstoff oder Kohlendioxid.

Die folgenden Beispiele sollen die Erfindung erläutern.

### Vergleichsbeispiel 1:

Es wurde zunächst aus SiO₂-Mikrokugeln mit einer Oberfläche von 200 m²/g mit 10 Gew.-% Magnesiumstearat als Bindemittel ein Träger hergestellt. Der fertige Träger enthielt 0,4 Gew.-% Mg. Die Oberfläche betrug 186 m²/g und das Porenvolumen 0,8 ml/g, wobei 78 % des Porenvolumens von Poren mit einem Radius von 70 - 100 Å und 16 % des Porenvolumens von Poren mit einem Radius von 200 - 3000 Å gebildet wurden. Die Trägerteilchen hatten die Form von Zylindern mit gewölbten Stirnflächen (6 mm Durchmesser und 6 mm Höhe).

100 g des Trägers wurden mit einer (dem Porenvolumen des Trägers entsprechenden) Lösung von 11,5 g Pd-Acetat, 10,0 g Cd-Acetat und 10,8 g K-Acetat in 66 ml Eisessig getränkt und bei 60°C unter Stickstoff bei einem Druck von 200 mbar bis zu einem Lösungsmittelrestgehalt von 2 Gew.-% getrocknet. Dies ergab eine Dotierung von 2,3 Gew.-% Pd, 1,8 Gew.-% Cd und 2,0 Gew.-% K.

50 ml des fertigen, homogen imprägnierten Katalysators wurden in ein Reaktionsrohr von 8 mm Innendurchmesser und einer Länge von 1,5 m eingefüllt.

Bei einem Druck von 8 bar am Reaktoreingang und einer Katalysatortemperatur von 150°C wurde das aus 27 Vol.-% Ethylen, 55 Vol.-% Stickstoff, 12 Vol.-% Essigsäure und 6 Vol.-% Sauerstoff bestehende Reaktionsgas übergeleitet. Die Ergebnisse sind aus der Tabelle ersichtlich.

### Vergleichsbeispiel 2:

Der gleiche Träger wie im Vergleichsbeispiel 1 wurde mit einer Lösung von 2,6 g Pd-Chlorid und 3,3 g Cd-Chlorid in 83 ml Wasser imprägniert. Nach der Trocknung wurden 1,5 g NaOH in 83 ml Wasser zugegeben und 6 Stunden gerührt. Nach 16-stündigem Stehen bei Raumtemperatur wurde mit viel Wasser gewaschen und anschließend getrocknet. Danach wurde mit einer Lösung von 12,5 g K-Acetat in 83 ml Wasser imprägniert und erneut getrocknet. Man erhielt einen homogen imprägnierten Katalysator mit einer Dotierung von 1,5 Gew.-% Pd, 2,0 Gew.-% Cd und 5,0 Gew.-% K. Die Ausprüfung erfolgte wie in Vergleichsbeispiel 1.

### Vergleichsbeispiel 3:

Der gleiche Träger wie in Vergleichsbeispiel 1 wurde mit einer Lösung von 2,8 g Na₂PdCl₄ und 0,7 g HAuCl₄ imprägniert und getrocknet. Nach der Fällung mit 1,1 g wäßriger NaOH wurde mit viel Wasser gewaschen und getrocknet. Nach Imprägnierung mit einer wäßrigen Lösung von 7,0 g K-Acetat wurde erneut getrocknet. Der Katalysator enthielt 1,0 Gew.-% Pd, 0,4 Gew.-% Au und 2,8 Gew.-% K. Eine Schale war nicht vorhanden, d.h. der Katalysator war wieder homogen imprägniert. Die Austestung erfolgte in einem Berty-Reaktor bei 154°C mit einem Gasgemisch aus 8 % O₂, 37,5 % C₂H₄, 15,7 % HOAc und 38,8 % N₂.

### Beispiel 1:

Der Katalysator wurde wie in Vergleichsbeispiel 2 hergestellt, mit der Ausnahme, daß der Träger vor der Imprägnierung mit Palladium- und Cadmiumchlorid mit 10 %iger Salzsäure für 14 Stunden bei Raumtemperatur in Kontakt gebracht und anschließend unter fließendem Wasser chloridfrei gewaschen und dann getrocknet wurde. Man erhielt einen Schalenkatalysator. Der Restgehalt an Bindemittel-Kationen (Mg) betrug 0,01 %. Die Ausprüfung erfolgte wie in Vergleichsbeispiel 1.

### Beispiel 2:

Der Katalysator wurde wie in Vergleichsbeispiel 3 hergestellt, mit der Ausnahme, daß der Träger vor der Imprägnierung mit Palladium- und Goldsalzen wie in Beispiel 1 mit Salzsäure gewaschen, dann mit Wasser chloridfrei gewaschen und getrocknet wurde. Die Ausprüfung des erhaltenen Schalenkatalysators erfolgte wie in Vergleichsbeispiel 3.

**Tabelle**

| | Vergleichsbeispiel | | | Beispiel | |
|---|---|---|---|---|---|
| | 1 | 2 | 3 | 1 | 2 |
| RZA (g Vinylacetat pro Liter Katalysator und pro Stunde) | 773 | 440 | 657 | 752 | 698 |
| spezifische Leistung (g Vinylacetat pro g Edelmetall und pro Stunde) | 67,2 | 58,7 | 93,9 | 100,3 | 99,7 |
| Verbrennung (%) | 14 | 16 | 12,5 | 7,8 | 11,2 |
| Ethylacetatgehalt (ppm) | 260 | 260 | 314 | 120 | 222 |
| "RZA" bedeutet die Raum-Zeit-Ausbeute "Verbrennung" bedeutet den Prozentsatz des umgesetzten Ethylens, der in CO₂ umgewandelt wird; "Ethylacetatgehalt" bezieht sich auf den Gehalt der kondensierten Reaktionsprodukte an Ethylacetat. | | | | | |

## Patentansprüche

1. Verfahren zur Herstellung eines Katalysators, der Palladium und/oder dessen Verbindungen und Alkaliverbindungen, sowie zusätzlich Cadmiumverbindungen und/oder Gold und/oder dessen Verbindungen auf Trägerteilchen enthält, die aus SiO₂ oder einem SiO₂-Al₂O₃-Gemisch mit Hilfe eines Bindemittels, bestehend aus einem oder mehreren Li-, Mg-, Al-, Zn-, Fe- oder Mn-Salzen einer C₂-C₂₆-Carboxylsäure, gepreßt und anschließend in sauerstoffhaltigen Gasen bei 500 - 900°C für eine Dauer von 0,25 - 5 Stunden geglüht wurden und danach eine Oberfläche von 50 - 250 m²/g und ein Porenvolumen von 0,4 - 1,2 ml/g bei einer Teilchengröße von 1 - 15 mm besitzen, wobei 5 - 20 % des Porenvolumens von Poren mit einem Radius von 200 - 3000 Å und 50 - 90 % des Porenvolumens von Poren mit einem Radius von 70 - 100 Å gebildet wird, dadurch gekennzeichnet, daß man die geglühten Trägerteilchen
a) mit einer Säure wäscht, die nicht mit SiO₂ oder SiO₂-Al₂O₃-Gemischen reagiert, bis von den Trägerteilchen keine weiteren Kationen des beim Pressen der Trägerteilchen eingesetzten Bindemittels abgegeben werden;
b) dann die Trägerteilchen mit Palladium, sowie Gold oder Cadmium imprägniert;
c) dann die imprägnierten Trägerteilchen mindestens solange mit einer Lösung einer Base in Kontakt bringt, bis die Dicke der dadurch auf den Trägerteilchen erzeugten Edelmetallschale sich nicht mehr wesentlich ändert;
d) dann die Trägerteilchen mit einer Alkaliverbindung imprägniert.

2. Verfahren zur Herstellung von Vinylacetat in der Gasphase aus Ethylen, Essigsäure und Sauerstoff oder sauerstoffhaltigen Gasen an einem Katalysator, der Palladium und/oder dessen Verbindungen und Alkaliverbindungen, sowie zusätzlich Cadmiumverbindungen und/oder Gold und/oder dessen Verbindungen auf Trägerteilchen enthält, die aus SiO₂ oder einem SiO₂-Al₂O₃-Gemisch mit Hilfe eines Bindemittels, bestehend aus einem oder mehreren Li-, Mg-, Al-, Zn-, Fe- oder Mn-Salzen einer C₂-C₂₆-Carboxylsäure, gepreßt und anschließend in sauerstoffhaltigen Gasen bei 500 - 900°C für eine Dauer von 0,25 - 5 Stunden geglüht wurden und danach eine Oberfläche von 50 - 250 m²/g und ein Porenvolumen von 0,4 - 1,2 ml/g bei einer Teilchengröße von 1 - 15 mm besitzen, wobei 5 - 20 % des Porenvolumens von Poren mit einem Radius von 200 - 3000 Å und 50 - 90 % des Porenvolumens von Poren mit einem Radius von 70-100 Å gebildet wird, dadurch gekennzeichnet, daß man die geglühten Trägerteilchen
a) mit einer Säure wäscht, die nicht mit SiO₂ oder SiO₂-Al₂O₃-Gemischen reagiert, bis von den Trägerteilchen keine weiteren Kationen des beim Pressen der Trägerteilchen eingesetzten Bindemittels abgegeben werden;
b) dann die Trägerteilchen mit Palladium, sowie Gold oder Cadmium imprägniert;
c) dann die imprägnierten Trägerteilchen mindestens solange mit einer Lösung einer Base in Kontakt bringt, bis die Dicke der dadurch auf den Trägerteilchen erzeugten Edelmetallschale sich nicht mehr wesentlich ändert;
d) dann die Trägerteilchen mit einer Alkaliverbindung imprägniert.

3. Verfahren nach Anspruch 1 oder 2, dadurch gekennzeichnet, daß man als Säure in Schritt a) Salzsäure, Schwefelsäure, Phosphorsäure oder Salpetersäure verwendet.

4. Verfahren nach Anspruch 1 oder 2, dadurch gekennzeichnet, daß man als Säure in Schritt a) Salzsäure verwendet.

5. Verfahren nach einem der Ansprüche 1 bis 4, dadurch gekennzeichnet, daß man als Base in Schritt c) ein Alkalihydroxid verwendet.

6. Verfahren nach einem der Ansprüche 1 bis 5, dadurch gekennzeichnet, daß man in Schritt d) eine Kaliumverbindung einsetzt.

7. Katalysator, der Palladium und/oder dessen Verbindungen und Alkaliverbindungen, sowie zusätzlich Cadmiumverbindungen und/oder Gold und/oder dessen Verbindungen auf Trägerteilchen enthält, die aus SiO₂ oder einem SiO₂-Al₂O₃-Gemisch mit Hilfe eines Bindemittels, bestehend aus einem oder mehreren Li-, Mg-, Al-, Zn-, Fe- oder Mn-Salzen einer C₂-C₂₆-Carboxylsäure, gepreßt und anschließend in sauerstoffhaltigen Gasen bei 500 - 900°C für eine Dauer von 0,25 - 5 Stunden geglüht wurden und danach eine Oberfläche von 50 - 250 m²/g und ein Porenvolumen von 0,4 - 1,2 ml/g bei einer Teilchengröße von 1 - 15 mm besitzen, wobei 5 - 20 % des Porenvolumens von Poren mit einem Radius von 200 - 3000 Å und 50 - 90 % des Porenvolumens von Poren mit einem Radius von 70 - 100 Å gebildet wird, dadurch erhältlich, daß man die geglühten Trägerteilchen
a) mit einer Säure wäscht, die nicht mit SiO₂ oder SiO₂-Al₂O₃-Gemischen reagiert, bis von den Trägerteilchen keine weiteren Kationen des beim Pressen der Trägerteilchen eingesetzten Bindemittels abgegeben werden;
b) dann die Trägerteilchen mit Palladium, sowie Gold oder Cadmium imprägniert;
c) dann die imprägnierten Trägerteilchen mindestens solange mit einer Lösung einer Base in Kontakt bringt, bis die Dicke der dadurch auf den Trägerteilchen erzeugten Edelmetallschale sich nicht mehr wesentlich ändert;
d) dann die Trägerteilchen mit einer Alkaliverbindung imprägniert.

8. Katalysator nach Anspruch 7, dadurch gekennzeichnet, daß man als Säure in Schritt a) Salzsäure, Schwefelsäure, Phosphorsäure oder Salpetersäure verwendet.

9. Katalysator nach Anspruch 7 oder 8, dadurch gekennzeichnet, daß man als Base in Schritt c) ein Alkalihydroxid verwendet.

10. Katalysator nach einem der Ansprüche 7 bis 9, dadurch gekennzeichnet, daß man in Schritt d) eine Kaliumverbindung einsetzt.

## Claims

1. A process for the preparation of a catalyst which contains palladium and/or compounds thereof and alkali metal compounds, and additionally cadmium compounds and/or gold and/or compounds thereof, on support particles which have been pressed from SiO₂ or an SiO₂-Al₂O₃ mixture with the aid of a binder comprising one or more Li, Mg, Al, Zn, Fe or Mn salts of a C₂-C₂₆-carboxylic acid and have subsequently been roasted in oxygen-containing gases at 500-900°C for a period of 0.25-5 hours, and thereafter have a surface area of 50-250 m²/g and a pore volume of 0.4-1.2 ml/g at a particle size of 1-15 mm, 5-20% of the pore volume being formed by pores having a radius of 200-3000 Å and 50-90% of the pore volume being formed by pores having a radius of 70-100 Å, which comprises
a) washing the roasted support particles with an acid which does not react with SiO₂ or SiO₂-Al₂O₃ mixtures, until no further cations of the binder employed during pressing of the support particles are released from the support particles;
b) then impregnating the support particles with palladium, and gold or cadmium;
c) then bringing the impregnated support particles into contact with a solution of a base at least until the thickness of the noble metal shell generated in this way on the support particles no longer changes substantially; and
d) then impregnating the support particles with an alkali metal compound.

2. A process for the preparation of vinyl acetate in the gas phase from ethylene, acetic acid and oxygen or oxygen-containing gases over a catalyst which contains palladium and/or compounds thereof and alkali metal compounds, and additionally cadmium compounds and/or gold and/or compounds thereof, on support particles which have been pressed from SiO₂ or an SiO₂-Al₂O₃ mixture with the aid of a binder comprising one or more Li, Mg, Al, Zn, Fe or Mn salts of a C₂-C₂₆-carboxylic acid and have subsequently been roasted in oxygen-containing gases at 500-900°C for a period of 0.25-5 hours, and thereafter have a surface area of 50-250 m²/g and a pore volume of 0.4-1.2 ml/g at a particle size of 1-15 mm, 5-20% of the pore volume being formed by pores having a radius of 200-3000 Å and 50-90% of the pore volume being formed by pores having a radius of 70-100 Å, which comprises
a) washing the roasted support particles with an acid which does not react with SiO₂ or SiO₂-Al₂O₃ mixtures, until no further cations of the binder employed during pressing of the support particles are released from the support particles;
b) then impregnating the support particles with palladium, and gold or cadmium;
c) then bringing the impregnated support particles into contact with a solution of a base at least until the thickness of the noble metal shell generated in this way on the support particles no longer changes substantially; and
d) then impregnating the support particles with an alkali metal compound.

3. The process as claimed in claim 1 or 2, wherein hydrochloric acid, sulfuric acid, phosphoric acid or nitric acid is used as the acid in step a).

4. The process as claimed in claim 1 or 2, wherein hydrochloric acid is used as the acid in step a).

5. The process as claimed in one of claims 1 to 4, wherein an alkali metal hydroxide is used as the base in step c).

6. The process as claimed in one of claims 1 to 5, wherein a potassium compound is employed in step d).

7. A catalyst which contains palladium and/or compounds thereof and alkali metal compounds, and additionally cadmium compounds and/or gold and/or compounds thereof, on support particles which have been pressed from SiO₂ or an SiO₂-Al₂O₃ mixture with the aid of a binder comprising one or more Li, Mg, Al, Zn, Fe or Mn salts of a C₂-C₂₆-carboxylic acid and have subsequently been roasted in oxygen-containing gases at 500-900°C for a period of 0.25-5 hours, and thereafter have a surface area of 50-250 m²/g and a pore volume of 0.4-1.2 ml/g at a particle size of 1-15 mm, 5-20% of the pore volume being formed by pores having a radius of 200-3000 Å and 50-90% of the pore volume being formed by pores having a radius of 70-100 Å, obtainable by
a) washing the roasted support particles with an acid which does not react with SiO₂ or SiO₂-Al₂O₃ mixtures, until no further cations of the binder employed during pressing of the support particles are released from the support particles;
b) then impregnating the support particles with palladium, and gold or cadmium;
c) then bringing the impregnated support particles into contact with a solution of a base at least until the thickness of the noble metal shell generated in this way on the support particles no longer changes substantially; and
d) then impregnating the support particles with an alkali metal compound.

8. A catalyst as claimed in claim 7, wherein hydrochloric acid, sulfuric acid, phosphoric acid or nitric acid is used as the acid in step a).

9. A catalyst as claimed in claim 7 or 8, wherein an alkali metal hydroxide is used as the base in step c).

10. A catalyst as claimed in one of claims 7 to 9, wherein a potassium compound is employed in step d).

## Revendications

1. procédé de préparation d'un catalyseur contenant du palladium et/ou ses composés et des composés alcalins, ainsi que de plus des composés du cadmium et/ou de l'or et/ou ses composés sur des particules de support, qui sont comprimées à partir de SiO₂ ou d'un mélange SiO₂-Al₂O₃ à l'aide d'un agent liant, consistant en un ou plusieurs sels de Li, Mg, Al, Zn, Fe ou Mn d'un acide carboxylique en C₂-C₂₆, et sont ensuite recuites à une température comprise entre 500 et 900°C dans un gaz contenant de l'oxygène pendant une période comprise entre 0,25 heure et 5 heures et possèdent ensuite une surface comprise entre 50 et 250 m²/g et un volume poreux compris entre 0,4 et 1,2 ml/g avec une granulométrie comprise entre 1 et 15 mm, 5 à 20 % du volume poreux étant formé de pores ayant un rayon compris entre 200 et 3000 Å et 50 à 90 % du volume poreux étant constitué de pores ayant un rayon compris entre 70 et 100 Å, procédé qui est caractérisé en ce que,
a) on lave les particules de support recuites avec un acide, ne réagissant pas avec SiO₂ ou un mélange SiO₂-Al₂O₃, jusqu'à ce que plus aucun cation de l'agent liant introduit lors du moulage par compression des particules de support ne soit éliminé des particules de support;
b) on imprègne ensuite les particules de support avec du palladium, ainsi que de l'or ou du cadmium;
c) on met ensuite les particules de support imprégnées au contact d'une solution d'une base au moins jusqu'à ce que l'épaisseur de la couche de métal précieux générée sur les particules de support ne se modifie plus substantiellement;
d) on imprègne ensuite les particules de support avec un composé alcalin.

2. Procédé de préparation d'acétate de vinyle en phase gazeuse à partir d'éthylène, d'acide acétique et d'oxygène ou de gaz contenant de l'oxygène sur un catalyseur, qui contient du palladium et/ou ses composés et des composés alcalins, ainsi que de plus des composés du cadmium et/ou de l'or et/ou ses composés sur des particules de support, qui sont comprimées à partir de SiO₂ ou d'un mélange de SiO₂-Al₂O₃ à l'aide d'un agent liant comprenant un ou plusieurs sels de Li, Mg, Al, Zn, Fe ou Mn d'un acide carboxylique en C₂-C₂₆ et ensuite que l'on recuit dans un gaz contenant de l'oxygène à une température comprise entre 500 et 900°C pendant une durée comprise entre 0,25 et 5 heures possédant ensuite une surface comprise entre 50 et 250 m²/g et un volume poreux compris entre 0,4 et 1,2 ml/g avec une granulométrie comprise entre 1 et 15 mm, 5 à 20 % du volume poreux étant formés de pores ayant un rayon compris entre 200 et 3000 Å et 50 à 90 % du volume poreux étant formés de pores ayant un rayon compris entre 70 et 100 Å, caractérisé en ce que les particules de support recuites
a) sont lavées avec un acide, ne réagissant pas avec SiO₂ ou un mélange de SiO₂-Al₂O₃, jusqu'à ce que aucun autre cation de l'agent liant utilisé lors du compactage des particules de support ne soit éliminé des particules de support ;
b) ensuite les particules de support sont imprégnées par du palladium, ainsi que par de l'or ou du cadmium ;
c) ensuite les particules de support imprégnées sont mises au contact d'une solution d'une base au moins jusqu'à ce que l'épaisseur de la couche de métal précieux engendrée sur les particules de support ne varie plus substantiellement ;
d) les particules de support sont ensuite imprégnées par un composé alcalin.

3. Procédé selon la revendication 1 ou 2, caractérisé en ce que l'acide utilisé dans l'étape a) est l'acide chlorhydrique, l'acide sulfurique, l'acide phosphorique ou l'acide nitrique.

4. Procédé selon la revendication 1 ou 2, caractérisé en ce que l'acide utilisé dans l'étape a) est l'acide chlorhydrique.

5. Procédé selon l'une des revendications 1 à 4, caractérisé en ce que la base utilisée dans l'étape c) est un hydroxyde alcalin.

6. Procédé selon l'une des revendications 1 à 5, caractérisé en ce que on introduit dans l'étape d) un composé du potassium.

7. Catalyseur, qui contient du palladium et/ou ses composés et des composés alcalins, ainsi que de plus des composés du cadmium et/ou de l'or et/ou ses composés sur des particules de support, qui sont comprimées à partir de SiO₂ ou un mélange de SiO₂-Al₂O₃, à l'aide d'un agent liant contenant'un ou plusieurs sels de Li, Mg, Al, Zn, Fe d'un acide carboxylique en C₂-C₂₆, et ensuite qui sont recuits dans un gaz contenant de l'oxygène à une température comprise entre 500 et 900°C pendant une période comprise entre 0,25 et 5 heures et possèdent une surface comprise entre 50 et 250 m²/g et un volume poreux compris entre 0,4 et 1,2 ml/g avec une granulométrie comprise entre 1 et 15 mm 5 à 20 % du volume poreux étant formés de pores ayant un rayon compris entre 200 et 3000 Å et 50 à 90 % du volume poreux étant formés de pores ayant un rayon compris entre 70 et 100 Å, que l'on obtient par le moyen en ce que
a) on lave les particules recuites par un acide, qui ne réagit pas avec SiO₂ ou un mélange de SiO₂-Al₂O₃, jusqu'à ce que aucun autre cation de l'agent liant utilisé lors de la compression des particules de support ne soit éliminé des particules de support ;
b) ensuite les particules de support sont imprégnées par du palladium, ainsi que par de l'or ou du cadmium ;
c) ensuite les particules de support imprégnées sont mises en contact avec une solution d'une base au moins jusqu'à ce que l'épaisseur de la couche du métal précieux générée sur les particules de support ne varient plus substantiellement ;
d) ensuite les particules de support sont imprégnées par un composé alcalin.

8. Catalyseur selon la revendication 7, caractérisé en ce que l'on utilise comme acide de l'étape a) de l'acide chlorhydrique, de l'acide sulfurique, de l'acide phosphorique ou de l'acide nitrique.

9. Catalyseur selon la revendication 7 ou 8, caractérisé en ce que l'on utilise comme base pour l'étape c) un hydroxyde alcalin.

10. Catalyseur selon les revendications 7 à 9, caractérisé en ce que l'on introduit dans l'étape d) un composé du potassium.
